# FASCICULE DE BREVET EUROPEEN

(11) **EP 1 406 651 B1**
(45) Date de publication et mention de la délivrance du brevet: **30.04.2008**
(21) Numéro de dépôt: 02767552.9
(22) Date de dépôt: 08.07.2002
(51) Int. Cl.: A61K 38/39, A61K 38/07, C07K 5/103, C07K 14/78, A61P 17/00, C07K 5/10, A61K 8/64

(54) **PEPTIDE ET L'UTILISATION COSMETIQUE OU DERMATOLOGIQUE POUR AUGMENTER L'ADHESION ENTRE LES CELLULES CUTANEES**
PEPTID UND DESSEN KOSMETISCHE ODER DERMATOLOGISCHE VERWENDUNG ZUR VERBESSERUNG DER HAFTUNG ZWISCHEN HAUTZELLEN
COSMETIC OR DERMATOLOGICAL USE OF A PEPTIDE FOR PROMOTING ADHESION BETWEEN SKIN CELLS

(30) Priorité: 13.07.2001 FR 0109361
(43) Date de publication de la demande: 14.04.2004
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR)
(86) Numéro de dépôt international: PCT/FR2002/002379
(87) Numéro de publication internationale: WO 2003/008438

(56) Documents cités:
- WO-A-00/77042
- DATABASE WPI Section Ch, Week 198649 Derwent Publications Ltd., London, GB; Class D21, AN 1986-322208 XP002197936 & JP 61 238712 A (SHISEIDO CO LTD), 24 octobre 1986 (1986-10-24)
- MOYANO, JOSE V. ET AL: "Fibronectin Type III5 repeat contains a novel cell adhesion sequence, KLDAPT, which binds activated.alpha.4.beta.1 and.alpha.4.beta.7 integrins" J. BIOL. CHEM. (1997), 272(40), 24832-24836, XP002197935
- WATT F.M.: 'Role of integrins in regulating epidermal adhesion, growth and differentiation' THE EMBO JOURNAL vol. 21, no. 15, 2002, pages 3919 - 3926

### SEQUENCE LISTING

<110> VINCIENCE SA
<120> Cosmetic or dermatological use of peptides for promoting adhesion between skin cells.
<140> PCT/FR 02/02379
   <141> 2001-07-13
<160> 10
<170> PatentIn version 3.1
<210> 1
   <211> 6
   <212> PRT
   <213> Artificial
<400> 1
   Lys Leu Asp Ala Pro Thr
   1 5
<210> 2
   <211> 4
   <212> PRT
   <213> Artificial
<400> 2
   Leu Asp Ala Pro
   1
<210> 3
   <211> 5
   <212> PRT
   <213> Artificial
<220>
   <221> MISC_FEATURE
   <222> (1)..(1)
   <223> Xaa = any amino acid
<400> 3
   Xaa Leu Asp Ala Pro
   1 5
<210> 4
   <211> 6
   <212> PRT
   <213> Artificial
<220>
   <221> MISC_FEATURE
   <222> (1)..(6)
   <223> Xaa = any amino acid
<400> 4
   Xaa Leu Asp Ala Pro Xaa
   1 5
<210> 5
   <211> 7
   <212> PRT
   <213> Artificial
<220>
   <221> MISC_FEATURE
   <222> (1)..(7)
   <223> Xaa = any amino acid
<400> 5
   Xaa Leu Asp Ala Pro Xaa Xaa
   1 5
<210> 6
   <211> 6
   <212> PRT
   <213> Artificial
<220>
   <221> MISC_FEATURE
   <222> (1)..(2)
   <223> Xaa = any amino acid
<400> 6
   Xaa Xaa Leu Asp Ala Pro
   1 5
<210> 7
   <211> 7
   <212> PRT
   <213> Artificial
<220>
   <221> MISC_FEATURE
   <222> (1)..(7)
   <223> Xaa = any amino acid
<400> 7
   Xaa Xaa Leu Asp Ala Pro Xaa
   1 5
<210> 8
   <211> 8
   <212> PRT
   <213> Artificial
<220>
   <221> MISC_FEATURE
   <222> (1)..(8)
   <223> Xaa = any amino acid
<400> 8
   Xaa Xaa Leu Asp Ala Pro Xaa Xaa
   1 5
<210> 9
   <211> 5
   <212> PRT
   <213> Artificial
<220>
   <221> MISC_FEATURE
   <222> (5)..(5)
   <223> Xaa = any amino acid
<400> 9
   Leu Asp Ala Pro Xaa
   1 5
<210> 10
   <211> 6
   <212> PRT
   <213> Artificial
<220>
   <221> MISC_FEATURE
   <222> (5)..(6)
   <223> Xaa = any amino acid
<400> 10
   Leu Asp Ala Pro Xaa Xaa
   1 5

## Description

La présente invention se rapporte à l'utilisation pour la préparation d'une composition cosmétique ou dermatologique, d'une quantité efficace de peptide de séquence Lys-Leu-Asp-Ala-Pro-Thr, où les acides aminés peuvent être sous la forme L, D ou DL ; le peptide ou la composition étant destinés à :
- Augmenter l'adhésion entre les cellules cutanées,
- Traiter de manière curative et/ou préventive les manifestations cutanées du vieillissement (d'origine physiologique et solaire) et améliorer l'aspect de la peau.

Le vieillissement cutané est un phénomène complexe qui est dû à de nombreux facteurs endogènes et exogènes. Cliniquement, on observe l'apparition de rides et ridules, une perte de l'élasticité cutanée, un relâchement des tissus cutanés et sous-cutanés...

De nombreuses voies de recherche sont proposées pour lutter contre le vieillissement, parmi lesquelles la protection contre l'environnement (soleil, pollutions...), l'activation de la régénération cellulaire, le renforcement de la matrice extracellulaire (collagène et élastine). Récemment, des études ont montré l'importance de l'adhésion kératinocytes-jonction dermo-épidermique dans le traitement des peaux âgées.

De façon générale, en augmentant l'adhésion des cellules entre elles, et l'adhésion entre les cellules et la matrice extra-cellulaire, il est possible de prévenir, voire de traiter le relâchement de la peau.

Cette dernière voie de recherche, prometteuse, est encore peu étudiée, alors que le rapprochement cellule-matrice ne peut que favoriser les échanges que l'on sait maintenant nombreux entre la cellule et son milieu environnant complexe.

La fibronectine est une protéine synthétisée par les fibroblastes et les kératinocytes, elle est présente dans la matrice extracellulaire où elle forme un vaste réseau d'adhésion entre les cellules et les composants de la matrice extracellulaire.

Elle joue un rôle fondamental dans une large panoplie d'activités biologiques ; notamment, elle joue un rôle essentiel dans l'acquisition de la structure de faisceaux de collagène et dans l'acquisition de propriétés biomécaniques de la peau en participant à l'adhésion des cellules à la matrice extracellulaire. Cette bonne adhésion est en relation directe avec une organisation tridimensionnelle optimale du tissu cutané, caractéristique d'une peau jeune et saine. La fibronectine est, de ce fait, un acteur majeur dans la migration et la communication cellulaire.

Très étudiée pour ses nombreuses fonctions, la fibronectine est aussi abondamment utilisée en cosmétique bien que sa structure de protéine (taille importante et charge) grève son efficacité au niveau cutané. Ainsi, le brevet JP61238712, décrit des compositions cosmétiques à base de substances augmentant l'adhésion cellulaire, telles que la fibronectine ou la laminine. Cette invention a pour but d'améliorer la stabilité et d'éviter le dépôt de fibronectine ou de laminine, dans les compositions, à basse température, par l'ajout d'une quantité spécifique de mucopolysaccharides.

Comme il est souvent le cas dans ce type de problématique, la recherche dermatologique se tourne vers des séquences peptidiques, donc plus courtes, qui pourraient mimer les activités de la fibronectine. L'industrie cosmétique, récemment sensibilisée à l'utilisation des peptides en biologie cutanée (séquences dérivées de l'alpha-MSH, de certains neuropeptides comme le NPY et la SP...), est à la recherche de peptides ayant une activité forte au niveau cutané. On constate donc que subsiste le besoin d'un nouveau dérivé de la fibronectine, ayant une action sur l'adhésion entre les cellules cutanées.

Dans un tout autre domaine que la dermatologie, Moyano J. V. et al (J. Biol. Chem., 1997, 272(40)), 24832-24836 ont montré que la séquence peptidique Lys-Leu-Asp-Ala-Pro-Thr (KLDAPT), présente au sein du motif répété de type III5 de la fibronectine, avait la propriété de se lier spécifiquement à des récepteurs présents à la surface de certaines cellules lymphoïdes activées.

Or, la demanderesse a trouvé, de façon surprenante et inattendue, qu'une quantité efficace du peptide de séquence Lys-Leu-Asp-Ala-Pro-Thr a une action sur l'adhésion entre les cellules cutanées. A la connaissance de la demanderesse, il n'a jamais été décrit dans l'art antérieur l'utilisation d'un peptide de séquence Lys-Leu-Asp-Ala-Pro-Thr en cosmétique.

Ainsi, l'invention a pour objet premier l'utilisation pour la préparation d'une composition cosmétique ou dermatologique, d'une quantité efficace de peptide de séquence Lys-Leu-Asp-Ala-Pro-Thr, où les acides aminés peuvent être sous la forme L, D ou DL ; le peptide ou la composition étant destinés à augmenter l'adhésion entre les cellules cutanées.

Par le terme "adhésion entre les cellules cutanées", on entend d'une part l'adhésion entre les cellules cutanées et la matrice extracellulaire, et d'autre part l'adhésion des cellules entre elles.

Par l'expression "augmenter l'adhésion entre les cellules cutanées", on entend le fait de stimuler l'expression de protéines visant à renforcer l'adhésion cellulaire, ainsi qu'à enrichir la matrice extracellulaire en protéines qui la compose.

Ainsi, le peptide ou la composition selon l'invention permettent d'augmenter l'expression des protéines de la matrice extracellulaire. On peut citer, à titre d'exemple de protéines de la matrice extracellulaire, des protéines telles que le collagène, la fibronectine, la laminine ou encore l'élastine. Toutes ces protéines sont constitutives de la matrice et y occupent un rôle fondamental, elles jouent notamment un rôle important dans l'adhésion.

Plus particulièrement, le peptide ou la composition selon l'invention permettent d'augmenter la synthèse de laminine, notamment, ils permettent d'augmenter la synthèse de la laminine-5. Cette protéine interagit avec les intégrines du pole basal des kératinocytes, permettant ainsi d'ancrer les cellules sur le réseau bidimensionnel de la jonction dermo-épidermique.

L'adhésion cellulaire s'effectue, notamment, grâce à des glycoprotéines membranaires, les intégrines. Ces protéines s'associent à diverses molécules de la matrice extracellulaire comme la fibronectine ou la laminine. Elles sont très impliquées dans l'adhésion des kératinocytes à la matrice extra-cellulaire, dans les liaisons cellule-cellule et dans la cohésion de l'assise basale de l'épiderme. Ainsi, une augmentation de la capacité d'adhérence des cellules cutanées peut se traduire comme une augmentation de l'expression des intégrines.

Une autre caractéristique de l'invention est donc l'utilisation du peptide ou de la composition afin d'augmenter la synthèse d'intégrines.

Par ailleurs, le renforcement de l'adhésion cellulaire permet de préserver la structure des fibres de collagène et de combattre l'atrophie cutanée inhérente au vieillissement, notamment au vieillissement photo-induit. Le peptide selon l'invention agit de façon très ciblée sur la cohésion, la communication et l'organisation tridimensionnelle du tissu cutané, favorisant ainsi la lutte contre la désorganisation structurelle due au vieillissement cutané.

Ainsi, l'invention a pour autre objet l'utilisation pour la préparation d'une composition cosmétique ou dermatologique, d'une quantité efficace de peptide de séquence Lys-Leu-Asp-Ala-Pro-Thr , où les acides aminés peuvent être sous la forme L, D ou DL ; le peptide ou la composition étant destinés à traiter de manière curative et/ou préventive les manifestations cutanées du vieillissement et à améliorer l'aspect de la peau.

Par l'expression "améliorer l'aspect de la peau", on entend tous les phénomènes qui sont susceptibles d'avoir pour conséquences une amélioration visuelle de l'état de la peau. La peau présentera une meilleure apparence ; elle sera, par exemple, beaucoup plus belle, ferme et/ou lisse. Toutes les petites imperfections seront diminuées ou supprimées. L'aspect papyracé de la peau sera, par exemple, atténué.

Par modifications cutanées du vieillissement on entend toutes modifications de l'aspect extérieur de la peau dues au vieillissement, qu'il soit d'origine physiologique ou solaire, comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets.

Le vieillissement de la peau se traduit, entre autres, par des altérations quantitatives et qualitatives des interactions entre les différents composants de la matrice extracellulaire, et résulte en l'apparition de rides et de ridules. Ces altérations sont particulièrement sévères dans les régions exposées aux UV. En effet, les fibroblastes exposés aux UV adhèrent plus faiblement à la fibronectine, et cela a notamment pour conséquence une dégradation des fibres de collagène.

Il se peut que pour des questions de résistance à la dégradation, il soit nécessaire d'utiliser selon l'invention une forme protégée du peptide. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées ; elles sont bien connues de l'homme du métier comme, par exemple, l'acylation ou l'acétylation de l'extrémité amino-terminale, ou l'amidation ou l'estérification de l'extrémité carboxy-terminale.

Ainsi, l'invention concerne une utilisation telle que définie précédemment caractérisée par le fait que le peptide est sous forme protégée ou non.

De préférence, on utilise une protection basée soit sur l'acylation de l'extrémité amino-terminale (N-terminale), soit sur l'estérification de l'extrémité carboxy-terminale (C-terminale), soit encore des deux.

Dans le domaine des acides aminés, la géométrie des molécules est telle qu'elles peuvent théoriquement se présenter sous la forme d'isomères optiques différents. Il existe en effet une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide, aussi bien des acides aminés sous forme d'isomères optiques dextrogyre ou lévogyre.

Ainsi, les acides aminés constituant le peptide selon l'invention, peuvent être sous configuration L- et D-, de manière préférentielle, les acides aminés sont sous forme L-. Le peptide selon l'invention peut donc être sous forme L-, D- ou DL-.

Le peptide, objet du présent brevet, peut être obtenu soit par synthèse chimique classique (en phase solide ou en phase homogène liquide), soit par synthèse enzymatique (Kullman et al., J. Biol. Chem. 1980, 225, 8234) à partir d'acides aminés constitutifs ou de leurs dérivés.

Le peptide de l'invention peut encore être obtenu par biotechnologie (utilisation d'un micro-organisme modifié ou non par génie génétique) ; c'est-à-dire que le peptide selon l'invention peut être aussi obtenu par fermentation d'une souche de bactéries modifiées ou non, ou encore par génie génétique.

Le peptide de l'invention peut aussi être obtenu à partir de protéines naturelles ; c'est-à-dire par extraction de protéines d'origine animale ou végétale, suivie d'une hydrolyse contrôlée qui libère les fragments peptidiques de taille moyenne et de petite taille dont il est question, avec la condition que les éléments libérés doivent contenir au moins la séquence Lys-Leu-Asp-Ala-Pro-Thr.

Il est possible, mais non nécessaire, pour réaliser l'invention d'extraire soit les protéines concernées d'abord et de les hydrolyser ensuite, soit d'effectuer l'hydrolyse d'abord sur un extrait brut et de purifier les fragments peptidiques ensuite.

D'autres procédés plus simples ou plus complexes peuvent être envisagés par l'homme du métier connaissant le métier de synthèse, d'extraction et de purification des protéines et des peptides.

Ainsi, le peptide selon l'invention peut être un peptide d'origine naturelle ou synthétique. Préférentiellement selon l'invention, le peptide est obtenu par synthèse chimique.

Selon un autre mode de réalisation avantageux de l'invention, le peptide précité est préalablement solubilisé dans un ou plusieurs solvants cosmétiquement ou pharmaceutiquement acceptables comme l'eau, le propylène glycol, le butylène glycol, les diglycols éthoxylés ou propoxylés, l'éthanol, le propanol ou l'isopropanol.

Selon encore un autre mode de réalisation avantageux de l'invention, le peptide précité est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

La composition selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage.

La composition selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains.

La quantité efficace de principe actif correspond à la quantité nécessaire pour obtenir le résultat désiré.

Pour donner un ordre de grandeur, dans les compositions cosmétiques ou dermatologiques de la présente invention, le peptide de séquence Lys-Leu-Asp-Ala-Pro-Thr est utilisé en une concentration représentant de 0,0005 à 500 ppm (p/p) et préférentiellement en une concentration représentant de 0,05 à 50 ppm (p/p).

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions pourront notamment se présenter sous forme de crèmes, émulsions huile-dans-eau, eau-dans-huile ou émulsions multiples, solutions, suspensions, ou encore poudres, adaptées à une application sur la peau, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, de façon connue, les adjuvants nécessaires à leur formulation, tels que solvants, épaississants, diluants, anti-oxydants, colorants, filtres, pigments, charges, conservateurs, parfums, absorbeurs d'odeur. Dans tous les cas, ces adjuvants, ainsi que leurs proportions, seront choisis de manière à ne pas nuire aux propriétés recherchées dans l'invention. Ces adjuvants peuvent, par exemple, correspondre à 0,01 à 20 % du poids total de la composition.

Lorsque la composition de l'invention est une émulsion, la phase grasse peut représenter de 5 à 80 % en poids et de préférence de 5 à 50 % en poids par rapport au poids total de la composition. Les émulsionnants et coémulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme de métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Aussi, un autre objet de l'invention consiste en une composition cosmétique ou dermatologique, caractérisée en ce qu'elle contient, dans un milieu cosmétiquement ou dermatologiquement acceptable, un peptide de Lys-Leu-Asp-Ala-Pro-Thr.

Dans les compositions de l'invention, le peptide de séquence Lys-Leu-Asp-Ala-Pro-Thr est utilisé en une concentration représentant de 0,0005 à 500 ppm (p/p) et préférentiellement en une concentration représentant de 0,05 à 50 ppm (p/p).

L'invention concerne enfin un procédé cosmétique pour le traitement des manifestations du vieillissement, consistant à appliquer, sur la peau ou les cheveux, la composition telle que définie précédemment.

D'autres avantages et caractéristiques de l'invention apparaîtront mieux à la lecture des exemples donnés à titre illustratif et non limitatif.

### Exemple 1 - Etude de la stabilité du peptide Lys-Leu-Asp-Ala-Pro-Thr.

Le peptide Lys-Leu-Asp-Ala-Pro-Thr a été analysé à une concentration de 10⁻⁴ M par HPLC sur une colonne de type C18, et avec un gradient linéaire eau/TFA 0,1 % - acétonitrile/TFA 0,1 %. Après 24 heures à différentes températures (25 °C, 37 °C et 60°C), aucune dégradation du peptide n'a été observée. De plus, après 8 jours à 25 °C, le peptide ne présente toujours pas de dégradation.

Des fibroblastes et des kératinocytes humains ont été cultivés pendant 24 heures à 37°C et à 5 % de CO2. Pendant cette période, les cellules vont libérer, dans le milieu de culture, de nombreuses enzymes de dégradation. Par la suite, le milieu de culture est retiré pour être mis en présence du peptide. Les résultats des analyses effectuées démontrent, qu'après 24 heures, le peptide ne présente quasiment pas de dégradation.

Un test est effectué en appliquant le peptide sur des fibroblastes ainsi que sur des kératinocytes humains en culture. Une analyse HPLC du milieu de culture révèle que la concentration en peptide décroît rapidement, c'est-à-dire en quelques heures.

Ces résultats, pris dans leur ensemble, suggèrent la possibilité d'une pénétration du peptide dans la cellule. Cette hypothèse est ensuite confortée par les tests d'efficacité exposés dans les exemples suivants.

### Exemple 2 - Effets du peptide de l'exemple 1 sur l'adhésion entre les cellules cutanées

L'étude est réalisée dans des micro-plaques de 96 puits sur des kératinocytes cultivés dans un incubateur à 37 °C et à 5 % de CO2.

Les puits de ces plaques sont prétraitées, pendant 12 heures, de manières différentes ; quatre conditions sont ainsi réalisées :
- Condition A : contrôle négatif, ne contenant pas de peptide,
- Condition B : incubée avec différents peptides, constitués de 3 à 25 acides aminés, à une concentration de 35 ppm,
- Condition C : incubée avec de la fibronectine à une concentration de 50 ppm,
- Condition D : incubée avec le peptide de séquence Lys-Leu-Asp-Ala-Pro-Thr à une concentration de 5 ppm.

Après 3 heures de contact avec les kératinocytes, les puits sont totalement remplis de milieu, hermétiquement fermés, retournés et agités sur un agitateur tridimensionnel pendant 20 minutes. Puis les plaques sont vidées et le milieu restant est aspiré. Ensuite, 100 µl de MTT à 1 mg/ml sont ajoutés par puits et laissés pendant 3 heures à 37 °C et à 5 % de CO₂. La solution est enfin retirée, 100 µl de DMSO sont ensuite ajoutés. Une lecture de la DO est faite à 560 nm contre 630 nm.

Les résultats présentent les différentes Densités Optiques (DO) obtenues en fonctions des différentes conditions réalisées. La DO est proportionnelle à la quantité de cellules viables, c'est-à-dire qui ont adhéré aux micro-plaques.

| Conditions | A | B | C | D |
|---|---|---|---|---|
| D.O. | 0,490 | 0,490 | 0,770 | 0,78 |

Ces résultats démontrent, après seulement 3 heures de contact avec les kératinocytes, le peptide de séquence Lys-Leu-Asp-Ala-Pro-Thr apporte une très bonne adhésion cellulaire, semblable à celle de la fibronectine, bien qu'utilisé en concentration plus faible que la fibronectine (10 fois plus faible).

### Exemple 3 - Mises en évidence par immunofluorescence de l'effet du peptide de l'exemple 1 sur l'expression de la laminine-5 et sur l'expression des intégrines β1.

Le but de l'étude est de déterminer l'influence du peptide de l'exemple 1 sur la synthèse de laminine-5 et sur la synthèse d'intégrine β1, par les kératinocytes, par immunofluorescence.

L'immunofluorescence est une technique semi-quantitative qui permet d'apprécier le taux de chacune des protéines présente dans le cytoplasme cellulaire.

Des kératinocytes humains HaCat sont ensemencés dans des Labteks puis mis en culture pendant une nuit. Après rinçage avec du tampon HBSS, une composition contenant une concentration de 10-6 M du peptide de séquence Lys-Leu-Asp-Ala-Pro-Thr, ou une composition contrôle ne contenant pas de peptide, est ajoutée. Les cellules sont ensuite incubées durant 48 heures. Après élimination des surnageants et rinçage des cultures, les cellules sont fixées avec du paraformaldhéydes pendant 30 minutes à 4°C puis rincées avec du tampon PBS.

200µL d'anticorps anti-laminine-5 et/ou d'anticorps anti-intégrine β1 sont alors ajoutés. L'incubation dure 30 minutes à température ambiante. Les surnageants sont éliminés et les cellules sont rincées au PBS. 200µL d'anticorps secondaires, couplés à un marqueur fluorescent (la fluorescéine) sont ensuite additionnés. Après 30 minutes d'incubation à température ambiante, les surnageants sont éliminés et les cellules sont rincées au PBS.

Les lames sont alors montées puis examinées au microscope à fluorescence inversée. Les quantités de laminine ou d'intégrine synthétisées par les cellules sont proportionnelles à l'intensité de la fluorescence.

Les résultats obtenus démontrent que l'ajout de peptides dans le milieu de culture des kératinocytes a pour effet d'augmenter la synthèse de laminine-5 et/ou la synthèse des intégrines β1 par les cellules. Cette stimulation a été observée de manière importante.

En effet, lorsque les kératinocytes sont incubés en présence de la composition contenant le peptide, on assiste, au bout de 48 heures, à une augmentation de l'intensité de la fluorescence traduisant ainsi une stimulation de la synthèse de la laminine-5 et/ou une stimulation de la synthèse des intégrines β1 par les kératinocytes.

### Exemple 4 - Préparation de compositions

Ces compositions ont été obtenues par simple mélange des différents composants. Les quantités indiquées sont données en pourcentage de poids.

### 1-Emulsion huile dans eau

### Phase huileuse :

■ Montanov 68 (Cetearyl Alcohol and Cetearyl Glucoside) 5.00 %
■ Huile de Jojoba 5.00 %
■ Huile de Vaseline 5.00 %
■ Isopropyl Palmitate 7.00 %

### Phase aqueuse :

■ Glycérine 5.00 %
■ Allantoïne 0.10 %
■ Peptide de l'exemple 1 1 ppm
■ Sepigel 305 (Polyacrylamide and C13-14 Isoparaffin and Laureth-7) 0.30 %
■ Conservateur 0.50 %
■ Parfum 0.50 %
■ Eau qsp 100 %

### 2 - Gel

■ Carbopol Ultrez 10 (sol. à 2%) 25.00 %
■ Triéthanolamine 0.50 %
■ Peptide de l'exemple 1 0.1 ppm
■ Conservateur 0.20 %
■ EDTA (séquestrant) 0.10 %
■ Parfum 0.50 %
■ Eau qsp 100 %

### 3 - Lotion

■ Mono Propylène Glycol 1.00 %
■ Allantoïne 0.30 %
■ Glycérine 1.00 %
■ Cetiol HE (PEG-7 Glyceryl Cocoate) 1.00 %
■ Peptide de l'exemple 1 10 ppm
■ Conservateur 0.20 %
■ Parfum 0.50 %
■ Eau qsp 100 %

## Revendications

1. Utilisation pour la préparation d'une composition cosmétique ou dermatologique, d'une quantité efficace de peptide de séquence Lys-Leu-Asp-Ala-Pro-Thr, où les acides aminés peuvent être sous la forme L, D ou DL ; les peptides ou la composition étant destinés à augmenter l'adhésion entre les cellules cutanées.

2. Utilisation pour la préparation d'une composition cosmétique ou dermatologique, d'une quantité efficace de peptide de séquence Lys-Leu-Asp-Ala-Pro-Thr, où les acides aminés peuvent être sous la forme L, D ou DL ; les peptides ou la composition étant destinés à traiter de manière curative et/ou préventive les manifestations cutanées du vieillissement et améliorer l'aspect de la peau.

3. Utilisation pour la préparation d'une composition cosmétique ou dermatologique, d'une quantité efficace de peptide de séquence Lys-Leu-Asp-Ala-Pro-Thr, où les acides aminés peuvent être sous la forme L, D ou DL ; les peptides ou la composition étant destinés à augmenter l'expression des protéines de la matrice extracellulaire.

4. Utilisation selon l'une quelconque des revendications précédentes **caractérisée en ce que** le peptide est modifié par acylation sur sa fonction N-terminale et/ou par estérification sur sa fonction C-terminale.

5. Utilisation selon l'une quelconque des revendications précédentes **caractérisée en ce que** le peptide est obtenu par synthèse chimique ou enzymatique à partir des acides aminés constitutifs ou de leurs dérivés ; ou est obtenu par hydrolyse ménagée de protéines naturelles ; ou bien est obtenu par biotechnologie.

6. Utilisation selon l'une quelconque des revendications précédentes, **caractérisée en ce que** le peptide est préalablement solubilisé dans un ou plusieurs solvants cosmétiquement ou pharmaceutiquement acceptables comme l'eau, le propylène glycol, le butylène glycol, les diglycols éthoxylés ou propoxylés, l'éthanol, le propanol ou l'isopropanol.

7. Utilisation selon l'une quelconque des revendications précédentes, **caractérisée en ce que** le peptide est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

8. Utilisation selon l'une quelconque des revendications précédentes, **caractérisée en ce que** le peptide est utilisé en une concentration représentant de 0,0005 à 500 ppm (p/p) et préférentiellement en une concentration représentant de 0,05 à 50 ppm (p/p).

9. Composition cosmétique ou dermatologique, **caractérisée en ce qu'**elle contient un peptide de séquence Lys-Leu-Asp-Ala-Pro-Thr, où les acides aminés peuvent être sous la forme L, D ou DL, tel que défini dans l'une des revendications 4 à 8.

10. Procédé cosmétique pour le traitement des manifestations du vieillissement, consistant à appliquer, sur la peau ou les cheveux, la composition telle que définie dans la revendication 9.

## Claims

1. Use for the preparation of a cosmetic or dermatological formulation, of an effective quantity of peptide having the sequence Lys-Leu-Asp-Ala-Pro-Thr, where the amino acids may be in L, D or DL form; the peptides or formulation being intended to increase adhesion between skin cells.

2. Use for the preparation of a cosmetic or dermatological formulation, of an effective quantity of peptide having the sequence Lys-Leu-Asp-Ala-Pro-Thr, where the amino acids may be in L, D or DL form; the peptides or formulation being intended to treat skin signs of ageing in a curative and/or preventive manner and improve the appearance of skin.

3. Use for the preparation of a cosmetic or dermatological formulation, of an effective quantity of peptide having the sequence Lys-Leu-Asp-Ala-Pro-Thr, where the amino acids may be in L, D or DL form; the peptides or formulation being intended to increase the expression of extracellular matrix proteins.

4. Use according to any of the above claims **characterised in that** the peptide is modified by means of acylation on the N-terminal function thereof and/or by means of esterification on the C-terminal function thereof.

5. Use according to any of the above claims **characterised in that** the peptide is obtained by means of chemical or enzymatic synthesis from constitutive amino acids or derivatives thereof; or is obtained by means of controlled hydrolysis of natural proteins; or is obtained by means of biotechnology.

6. Use according to any of the above claims, **characterised in that** the peptide is previously solubilised in one or more cosmetically or pharmaceutically acceptable solvents such as water, propylene glycol, butylene glycol, ethoxyl or propoxyl diglycols, ethanol, propanol or isopropanol.

7. Use according to any of the above claims, **characterised in that** the peptide is previously solubilised in a cosmetic or pharmaceutical excipient such as liposomes or adsorbed on powdery organic polymers, mineral substrates such as talcs and bentonites, and more generally solubilised in, or fixed on, any cosmetically or pharmaceutically acceptable vector.

8. Use according to any of the above claims, **characterised in that** the peptide is used at a concentration representing from 0.0005 to 500 ppm (w/w) and preferentially at a concentration representing from 0.05 to 50 ppm (w/w).

9. Cosmetic or dermatological formulation, **characterised in that** it contains a peptide having the sequence Lys-Leu-Asp-Ala-Pro-Thr, where the amino acids may be in L, D or DL form, as defined in any of claims 4 to 8.

10. Cosmetic method for the treatment of signs of ageing, consisting of applying, on the skin or hair, the formulation as defined in claim 9.

## Patentansprüche

1. Verwendung einer wirksamen Menge Peptide der Sequenz Lys-Leu-Asp-Ala-Pro-Thr für die Zubereitung einer kosmetischen oder dermatologischen Zusammensetzung, bei der die Aminosäuren eine L-, D- oder DL-Form aufweisen können; wobei die Peptide oder die Zusammensetzung dazu vorgesehen sind, die Haftung zwischen den Hautzellen zu erhöhen.

2. Verwendung einer wirksamen Menge Peptide der Sequenz Lys-Leu-Asp-Ala-Pro-Thr für die Zubereitung einer kosmetischen oder dermatologischen Zusammensetzung, bei der die Aminosäuren eine L-, D- oder DL-Form aufweisen können; wobei die Peptide oder die Zusammensetzung dazu vorgesehen sind, auf heilende und/oder präventive Weise die Alterungsmanifestationen der Haut zu behandeln und das Aussehen der Haut zu verbessern.

3. Verwendung einer wirksamen Menge Peptide der Sequenz Lys-Leu-Asp-Ala-Pro-Thr für die Zubereitung einer kosmetischen oder dermatologischen Zusammensetzung, bei der die Aminosäuren eine L-, D- oder DL-Form aufweisen können; wobei die Peptide oder die Zusammensetzung dazu vorgesehen sind, die Expression der Proteine der extrazellulären Matrix zu erhöhen.

4. Verwendung nach einem der vorhergehenden Ansprüche, **dadurch gekennzeichnet, dass** das Peptid durch Acylierung auf seiner N-terminalen Funktion und/oder durch Veresterung auf seiner C-terminalen Funktion verändert wird.

5. Verwendung nach einem der vorhergehenden Ansprüche, **dadurch gekennzeichnet, dass** das Peptid durch chemische oder enzymatische Synthese aus den konstitutiven Aminosäuren oder ihren Derivaten erhalten wird; oder durch gelenkte Hydrolyse von natürlichen Proteinen erhalten wird; oder auch durch Biotechnologie erhalten wird.

6. Verwendung nach einem der vorhergehenden Ansprüche, **dadurch gekennzeichnet, dass** das Peptid zuvor in einem oder mehreren kosmetisch oder pharmazeutisch zulässigen Lösemitteln wie z.B. Wasser, Propylenglykol, Butylenglykol, ethoxylierten oder propoxylierten Diglykolen, Ethanol, Propanol oder Isopropanol löslich gemacht wird.

7. Verwendung nach einem der vorhergehenden Ansprüche, **dadurch gekennzeichnet, dass** das Peptid vorher in einem kosmetischen oder pharmazeutischen Vektor wie z.B. den Liposomen löslich gemacht oder auf pulverigen organischen Polymeren und mineralischen Trägern wie z.B. Talkmineralien und Bentoniten absorbiert wird und insbesondere in jedem kosmetisch oder pharmazeutisch zulässigen Vektor löslich gemacht oder darauf befestigt wird.

8. Verwendung nach einem der vorhergehenden Ansprüche, **dadurch gekennzeichnet, dass** das Peptid in einer Konzentration von 0,0005 bis 500 ppm (p/p) und insbesondere in einer Konzentration von 0,05 bis 50 ppm (p/p) verwendet wird.

9. Kosmetische oder dermatologische Zusammensetzung, **dadurch gekennzeichnet, dass** sie ein Peptid der Sequenz Lys-Leu-Asp-Ala-Pro-Thr enthält, bei der die Aminosäuren eine L-, D- oder DL-Form aufweisen können, wie in einem der Ansprüche 4 bis 8 definiert.

10. Kosmetisches Verfahren für die Behandlung der Alterungsmanifestationen, umfassend das Auftragen der Zusammensetzung, wie in Anspruch 9 definiert, auf die Haut oder auf die Haare.
